# EUROPEAN PATENT SPECIFICATION

(11) **EP 0 915 946 B1**
(45) Date of publication and mention of the grant of the patent: **11.10.2000**
(21) Application number: 97932500.8
(22) Date of filing: 15.07.1997
(51) Int. Cl.: C11D 1/62, C07C 231/02

(54) **PROCESS FOR THE PREPARATION OF FABRIC SOFTENING ACTIVES AND PRODUCTS EMPLOYING SAME**
VERFAHREN ZUR HERSTELLUNG VON TEXTILWEICHMACHERZUSAMMENSETZUNGEN
PROCEDE DE PREPARATION D'AGENTS ACTIFS ADOUCISSEURS DE TISSUS ET PRODUITS DANS LESQUELS ILS SONT UTILISES

(30) Priority: 22.07.1996 US 22029 P
(43) Date of publication of application: 19.05.1999
(73) Proprietor: THE PROCTER & GAMBLE COMPANY, Cincinnati, Ohio 45202 (US)
(72) Inventor: WAHL, Errol, Hoffman, Cincinnati, OH 45242 (US); WAITE, Scott, William, Cincinnati, OH 45240 (US); SEVERNS, John, Cort, West Chester, OH 45069 (US); HARTMAN, Frederick, Anthony, Cincinnati, OH 45242 (US)
(74) Representative: Gault, Nathalie
(86) International application number: US9711726
(87) International publication number: WO9803618

(56) References cited:
- WO-A-96/21715
- US-A- 3 756 950
- US-A- 4 137 180
- US-A- 5 399 272

## Description

### FIELD OF THE INVENTION

The present invention relates to the preparation of quaternary fabric softening actives and products employing the same. In particular, the present invention relates to the addition of the chelant diethylenetriaminepentaacetic acid (DTPA) or ethylenediamine-N,N'-disuccinnic acid (EDDS) to the production of the softener active and to the preparation of fabric softener compositions, preferably clear or translucent comprising the softener actives produced by the process of the present invention.

### BACKGROUND OF THE INVENTION

Processes for the preparation of biodegradable quaternary fabric softener actives have been known. Such process involve the hydrolysis of a triglyceride to form a fatty acid, esterification of the fatty acid with an amine followed by quaternization of the fatty acid esters. Traditional fabric softening compositions, known as stable "dispersion" compositions, have not been particularly concerned with the discoloration of the softener active. Thus, prior art processes for the production of fabric softening actives have not been concerned with the color of the fabric softening active. However, with increasing efforts to develop clear fabric softening compositions to satisfy consumer demand, the need has arisen for fabric softening actives which have little or no color in the active.

Accordingly, there remains a need for a process for preparing a quaternary fabric softening active with reduced discoloration and malodor of the active.

### BACKGROUND ART

U.S. Patent No. 3,756,950 discloses the addition of chelants to fabric softening compositions to prevent yellowing of fabrics treated with the compositions. U.S. Patent No. 5,399,272 discloses clear liquid fabric softening compositions. U.S. Patent 5,525,245 also discloses clear liquid fabric softening compositions.

### SUMMARY OF THE INVENTION

This need is met by the present invention wherein a process for the preparation of a fabric softener active is provided wherein the chelant DTPA and/or EDDS is added to the process. The resulting softener active has reduced discoloration and malodor associated therewith.

According to a first embodiment of the present invention, a process for the preparation of a biodegradable fabric softening active is provided. The process comprises the steps of:
a) providing a source of triglyceride and reacting said source of triglyceride to form a mixture, said mixture comprising fatty acids having the formula R¹CO₂H, fatty acid esters having the formula R¹CO₂R", or mixtures thereof; wherein R¹ is C₁-C₂₂ alkyl, C₂-C₂₂ alkenyl, and mixtures thereof; R" is C₁-C₄ alkyl, C₂-C₄ monohydroxyalkyl, C₃-C₄ dihydroxyalkyl;
b) reacting said mixture from step (a) with one or more amines having the formula: 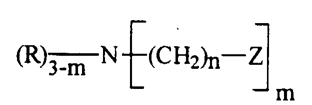 wherein R is C₁-C₆ alkyl, C₁-C₆ hydroxyalkyl, benzyl, or mixtures thereof; each Z is independently selected from the group consisting of -OH, -CHR'OH, -CH(OH)CH₂OH, -NH₂, and mixtures thereof, wherein R' is C₁-C₄ alkyl; m is an integer from 1 to 3; n is an integer from 1 to 4; to form a fabric softening active precursor mixture having the formula: 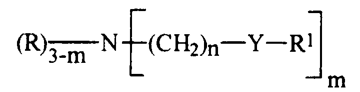 wherein R, R¹, m, and n are the same as above; Y has the formula: 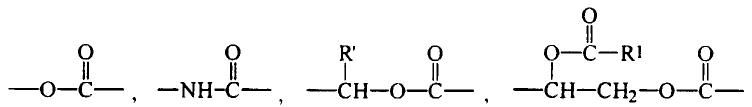 or mixtures thereof; wherein R¹ and R' are the same as above; and
c) quaternizing said fabric softening active precursor mixture from step (b) by reacting said mixture with a quaternizing agent of the formula RX, wherein R is the same as defined in step (b) and X is a softener compatible anion, forming a fabric softening active having the formula: 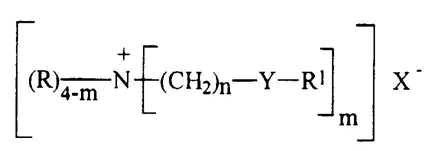 wherein R, R¹, X, Y, m, and n are the same as above;
provided at least step (c) is carried out in the presence of a chelating agent selected from the group consisting of diethylenetriaminepentaacetic acid, ethylenediamine-N,N'-disuccinnic acid, and mixtures thereof. Preferably, both steps b and c are carried out in the presence of chelant.

The step of reacting the source of triglyceride may further include reaction in the presence of the chelating agent and the step of forming the fabric softening active precursor mixture, Step (b), may further include reaction in the presence of a chelant. In fact, chelant may be added to each step of the present process, including optional hydrogenation steps described herein further below, to ensure a sufficient amount is present to reduce the formation of color and odor bodies in the event that chelant added in prior steps has been consumed.

The total amount of added chelating agent is preferably within the range of from 10 ppm, more preferably from about 100 ppm to 5000 ppm, more preferably to about 2500 ppm by weight of the formed softener active. The source of triglyceride can be any vegetable or animal source and is preferably selected from the group consisting of tallow, partially hydrogenated tallow, lard, partially hydrogenated lard, vegetable oils, partially hydrogenated vegetable oils, and mixtures thereof. More preferably, the vegetable oil or partially hydrogenated vegetable oil is selected from the group consisting of canola oil, partially hydrogenated canola oil, safflower oil, partially hydrogenated safflower oil, peanut oil, partially hydrogenated peanut oil, sunflower oil, partially hydrogenated sunflower oil, corn oil, partially hydrogenated corn oil, soybean oil, partially hydrogenated soybean oil, tall oil, partially hydrogenated tall oil, rice bran oil, partially hydrogenated rice bran oil, and mixtures thereof. Most preferably, the source of triglyceride is canola oil, partially hydrogenated canola oil, and mixtures thereof. The process may also include the step of adding from 0.001% to 2% by weight of the composition of an antioxidant compound to any or all of steps (a), (b) or (c). The products of the above process are new materials.

According to a second embodiment of the present invention, a process for the preparation of a fabric softening premix composition is provided. This method comprises preparing a fabric softening active as described above and mixing the quaternary fabric softener active, optionally containing a low molecular weight solvent, with a principal solvent having a ClogP of from 0.15 to 0.64 thereby forming a fabric softener premix. The premix may comprise from about 55% to about 85% by weight of fabric softening active and from about 10% to about 30% by weight of a principal solvent. The process may further comprise the step of adding a low molecular weight water soluble solvent selected from the group consisting of: ethanol, isopropanol, propylene glycol, 1,3-propanediol, propylene carbonate, hexylene glycol and mixtures thereof to the premix. Again, the process may also include the step of adding from 0.001% to 2% by weight of the composition of an antioxidant compound to any or all of steps (a), (b) or (c). The products of the above process are also new compositions.

According to another aspect of the present invention, a process for preparing a fabric softening composition is provided. This process comprises the steps of forming a premix as described above and the steps of forming a water seat by combining water and a mineral acid; and mixing the premix and the water seat with agitation to form a fabric softening composition. The process may further comprise one or more steps, including the steps of adjusting the viscosity of the fabric softening composition with the addition of a solution of calcium chloride, adding a chelating agent to the water seat and adding a perfume ingredient to the premix. The products of the above process are also new compositions.

Accordingly, it is an object of the present invention to produce a fabric softener active with reduced coloration and malodor of the active. It is another object of the present invention to produce a fabric softener active by adding a chelating agent to the process. It is yet another object of the present invention to form a fabric softener premix by adding a chelating agent to the process. It is still another object of the present invention to produce a fabric softener composition by adding a chelating agent to the process. These and other objects, features and advantages will be clear from the following disclosure, the examples and the appended claims.

All percentages, ratios and proportions herein are by weight, unless otherwise specified. All temperatures are in degrees Celsius (° C) unless otherwise specified. All documents cited are in relevant part, incorporated herein by reference.

### DETAILED DESCRIPTION OF THE INVENTION

### Preparation of Fabric Softening Actives

The fabric softening actives of the present invention are prepared from a source of triglyceride. The triglyceride source is preferably derived from tallow, partially hydrogenated tallow, lard, partially hydrogenated lard, vegetable oils and/or partially hydrogenated vegetable oils, such as, canola oil, safflower oil, peanut oil, sunflower oil, corn oil, soybean oil, tall oil, rice bran oil, etc. and mixtures of these oils.

One highly preferred triglyceride source which can be used to prepare the fabric softening compositions herein is canola oil. Canola oil is a mixture of triglycerides having an appropriate chain length distribution and degree of unsaturation of the respective fatty acyl groups. Canola oil is a particularly desirable starting product in accordance with the process of the present invention, for several reasons. In particular, its natural distribution of the chain lengths of the respective acyl groups has a notably high proportion of fatty acyl groups containing from about 12 to about 22 carbon atoms, thus avoiding the additional expense incurred when using other commercial sources of C₁₂-C₂₂ fatty acids as starting materials.

The triglyceride starting product can be hydrogenated, if desired, to convert di-unsaturated, tri-unsaturated, and polyunsaturated fatty acyl groups, particularly those containing from about 12 to about 22 carbon atoms, to their mono-unsaturated counterparts. It is normally desirable that hydrogenation of mono-unsaturated acyl groups is minimized and even completely avoided. Saturated fatty acyl groups can be obtained from normally saturated sources and mixed with unsaturated fatty acyl groups. In some useful mixtures of acyl groups, no more than about 10% of unsaturated C₁₂-C₂₂ acyl groups are hydrogenated to their saturated counterparts. For some products, hydrogenation of di-unsaturated and tri-unsaturated C₁₂-C₂₂ acyl groups is preferably maximized, consistent with minimal formation of saturated C₁₂-C₂₂ groups. For instance, tri-unsaturated fatty acyl groups can be completely hydrogenated without achieving complete hydrogenation of di-unsaturated acyl groups.

Hydrogenation of the triglyceride starting product which maximizes mono-unsaturated fatty acyl groups can be readily achieved by maintaining an appropriate balance of the conditions of the hydrogenation reaction. The process variables in the hydrogenation of triglycerides and the effects of altering such variables, are generally quite familiar to those of ordinary skill in this art. In general, hydrogenation of the triglyceride starting product can be carried out at a temperature ranging from about 170°C, preferably from about 185°C, to about 205°C, preferably to about 195°C. The other significant process variable is the pressure of hydrogen within the hydrogenation reactor. In general, this pressure should be maintained within a range (broadly stated) of from about 2 psig to about 20 psig, and more preferably between from 5 psig and about 15 psig.

Within these ranges of parameters, hydrogenation can be carried out with a particular view to the effects of these parameters. Lower hydrogen pressures in the reactor permit a greater degree of control of the reaction, particularly as to its selectivity. By "selectivity" is meant the hydrogenation of di-unsaturated and tri-unsaturated acyl groups without excessive hydrogenation of mono unsaturated fatty acyl groups. On the other hand, higher hydrogen pressures afford less selectivity. Selectivity can be desirable in certain instances.

Higher hydrogenation temperatures are associated with faster rates of hydrogenation and with greater selectivity of the hydrogenation. Conversely, lower hydrogenation temperatures are associated with less selectivity (i.e. increased hydrogenation of the mono unsaturated groups), and particularly with slower hydrogenation rates in general.

These considerations are also balanced with considerations of *cis/trans* stereochemistry with respect to unsaturation of the fatty acyl group. More specifically, the presence of unsaturation in the fatty acyl groups can lead to the formation of different *cis/trans* stereoisomers in the fatty acyl groups upon hydrogenation. There are two possible stereoisomeric configurations for double bonds when unsaturated fatty acyl groups are present; the *cis* form and the *trans* form. The presence of the *cis* form is preferred, as it is associated with a lower melting point of the eventual product and, thus with greater fluidity, and better low temperature phase stability of clear compositions. Thus, another reason that canola oil is a particularly preferred triglyceride starting product is that, as a naturally occurring material, the fatty acyl groups present in this triglyceride exhibit predominately the *cis* form. In the hydrogenation, higher hydrogen pressures are associated also with a decreased tendency of the fatty acyl group to undergo configuration change from the *cis* form to the undesirable *trans* form. Also, higher hydrogenation temperatures while favorable for some reasons are also associated with higher conversion of *cis* unsaturation to the *trans* form. Products exhibiting satisfactory properties can be obtained by appropriate control of the hydrogenation conditions so as to afford both selectivity and control of the stereochemical configurations of the product.

The hydrogenation is carried out in the presence of a suitable hydrogenation catalyst. Such catalysts are well known and commercially available. They generally comprise nickel, palladium, ruthenium or platinum, typically on a suitable catalyst support. A suitable catalyst is a nickel based catalyst such as sold by Engelhard under the trade designation "N-545"®.

In one variation, the hydrogenation is carried out to an end point at which hydrogenation of the di-unsaturation and tri-unsaturation in the triglyceride product is maximized, while formation of saturated acyl groups is minimized. The progress of the hydrogenation reaction toward the end point can readily be monitored by periodic measurement of the iodine value of the reaction mass. As the hydrogenation proceeds, the Iodine Value decreases. For example, the hydrogenation reaction can be discontinued when the Iodine Value reaches about 95.

A preferred variation of the present invention is to carry out the hydrogenation of the fatty acyl groups in the presence of a chelant. It has been surprisingly discovered that the presence of a chelant during hydrogenation reduces the amount of odor and color produced during processing. However, the chelant chosen must not be one which interferes with the hydrogenation process.

Other requirements for hydrogenation reactions are well known, such as the types of reactor, cooling means to maintain the desired temperature, the provision of means for agitation effective to provide adequate contact between the triglyceride and the hydrogen and catalyst, etc.

The triglyceride containing the desired acyl groups is reacted, typically by hydrolysis or transesterification, to obtain the desired fatty acyl groups as, e.g., the corresponding fatty acids and/or fatty acid esters. That is, the three ester bonds in the triglyceride are broken so that the hydrogenated combination of acyl groups is converted to a mixtures of fatty acids and/or esters having the same chain length distribution as in the acyl groups, and having the distribution of saturation and unsaturation provided by the hydrogenation reaction.

Hydrolysis can be carried out under any of the suitable conditions known in this art for hydrolysis of triglycerides into their fatty acid constituents. In general, the triglyceride is reacted with high temperature steam in a reactor, optionally in the presence of a catalyst, wherein the fatty acids are split off from glycerine, following which the steam is condensed to form an aqueous solution of glycerine and this solution is removed. Transesterification of the triglyceride can be carried out under any of the suitable conditions known in this art for transesterification of triglycerides into their fatty acid ester constituents. Hydrolysis may be carried out in the presence of a suitable chelant provided the chelant does not adversely affect the hydrolysis rate or products derived therefrom.

The mixture of fatty acids and/or fatty acid esters which is obtained in the reaction of the triglyceride step is then reacted with one or more amines having the formula: 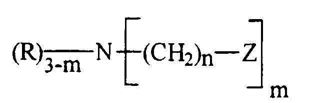 wherein R is C₁-C₆ alkyl, C₁-C₆ hydroxyalkyl, benzyl, or mixtures thereof; preferably methyl, each Z is independently selected from the group consisting of -OH, -CHR'OH, -CH(OH)-CH₂OH, -NH₂, and mixtures thereof, wherein R' is C₁-C₄ alkyl; m is an integer from 1 to 3; n is an integer from 1 to 4. A preferred starting amine has the formula R-N(CH₂CH₂OH)₂ of which triethanolamine is an preferred example. The result of this reaction of the fatty acyl feedstock (fatty acids, fatty acid esters, etc.) with an amine or amine mixture, is the formation of a fabric softening active precursor mixture.

In the case where R is -OH, -CHR'OH, and -CH(OH)CH₂OH, the reaction of the amine with the fatty acyl acids, fatty acyl esters, or mixtures thereof, is essentially an esterification reaction (or transesterification in the case where fatty acyl esters comprise all or part of the starting material). Esterification can be carried out under conventional esterification conditions, providing an acidic catalyst and providing for withdrawal of byproduct water of condensation. Preferably, a small amount, generally up to about 1.0 wt.% of the reactant (i.e. acids and amine), of hypophosphorous acid (HPPA) can be added to the esterification reaction mixture. HPPA is believed to catalyze the reaction and preserve, or even improve the color of the product obtained in this reaction.

In one embodiment of the present process, esterification is allowed to proceed completely such that all amine present is diesterified with fatty acids produced in the previous hydrolysis step. It is, however, sometimes desirable to produce a minor amount of the corresponding monoester.

The mixture of diesters, or mixture of diester and monoester components, as the case may be, is quaternized. Quaternization is carried out under conditions and with reactants generally familiar to those experienced in this field. The quaternizing agent has the formula RX, wherein R is preferably methyl, benzyl, or ethyl, and X is the anion as defined hereinabove. Preferably RX is methyl chloride, benzyl chloride, dimethyl sulfate, or diethyl sulfate. This quaternization step produces a mixture of fabric softening actives as described hereinabove. Preferably a solvent is used during the quaternization step, however, depending upon the choice of RX, the viscosity of the esterified fabric softener active precursor mixture, as well as other parameters germane to the desired final product admixture, the formulator may choose to conduct the quaternization without the use of solvent. Optionally, the quaternization reaction can be carried out in the presence of a chelant.

It is highly desirable that the compounds used herein are relatively free from unwanted impurities. Therefore, it is desirable to process the fatty acid source in ways that are known to eliminate such impurities, e.g., processing under atmospheres that are low in oxygen, separating unwanted materials by filtration, adsorption, distillation, etc., either before and/or after chemical modification by controlled hydrogenation. However, the purity of the materials is not part of the invention herein, which is equally applicable to less pure materials, the trade-off between purity and cost always being adjusted in light of the consumer's desires and needs.

Additional methods for the preparation of quaternary fabric softening compounds and actives are disclosed in U.S. Patent 5,524,433 the disclosure of which is herein incorporated by reference.

In the process of the present invention, a chelant is preferably added to the process to prevent yellowing or coloration of the fabric softener active and to reduce malodor. While not wishing to be bound by theory, it is believed that oxidation of the fatty chains in the softener active results in the formation of malodorous aldehydes and color bodies. This is particularly true when the fatty chains contain some degree of unsaturation, especially polyunsaturation. This oxidation of the fatty chains is catalyzed by the presence of transition metals that may be present in the triglyceride source, added as a catalyst in any hydrogenation step or metal contamination from process equipment. It is believed that the presence of a *trans*ition metal binding agent such as a chelant will reduce the oxidation of the fatty chains thereby reducing the formation of color bodies and malodorous aldehydes. Furthermore, by limiting the formation of color bodies, any fabric softening composition in which the fabric softening active is employed can be formulated as a truly clear product.

Accordingly, a chelant is added to the process of the present invention. The chelant may be added at any point in the formation of the fabric softener active from the point of reaction of the triglyceride to form a fatty acid and/or fatty acid ester to post addition of the chelant after quaternization of the fatty diester all of which is described in detail below. It is preferably to add the chelant material at the earliest possible point in time in the reaction of the source of triglyceride step and to add chelant at the beginning of every step following reaction of the triglyceride source to replenish catalyst levels in the product. Thus, chelant can be added during esterification, quaternization, and post added to the formed active. Of course, one of ordinary skill in the art will recognize that the catalyst may be added at any point in time between the reaction of triglyceride source and the post addition to the formed active. At a minimum, the chelant is present at the quaternization step.

The chelant according to the present invention is added at levels of from 10 ppm, more preferably from about 100 ppm, most preferably from about 250 ppm to 5,000 ppm, more preferably to about 2500 ppm, and most preferably to about 1500 ppm by weight of the formed active. If provided in salt form, it is preferred that the chelant be neutralized in solution to a pH of from about 4 to about 8 before addition to the process to ensure optimum performance from the added chelant.

The chelant employed in the present invention is selected from the group consisting of diethylenetriaminepentaacetic acid, ethylenediamine-N,N'-disuccinnic acid (EDDS), and mixtures thereof.

### FABRIC SOFTENING ACTIVES

The present invention relates to the preparation fabric softening actives as described hereinbefore. The fabric softening actives prepared are preferably diester quaternary fabric softening actives which are described in greater detail below. The fabric softening actives are preferably incorporated into compositions containing, as an essential component, from about 2%, preferably from about 13%, more preferably from about 15%, most preferably from about 19% to about 80%, preferably to about 75%, more preferably to about 70%, most preferably to about 65%, by weight of the composition, of the fabric softener actives, the fabric softener actives being selected from the compounds identified hereinafter, and mixtures thereof.

### Quaternary Ammonium Fabric Softening Active Compound (DEQA)

The fabric softening actives according to the present invention have the formula: 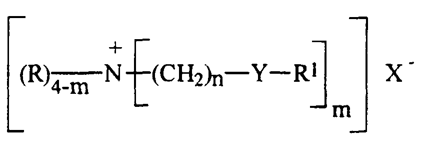 wherein each R is independently C₁-C₆ alkyl, C₁-C₆ hydroxyalkyl, benzyl, or mixtures thereof.

R¹ is C₁-C₂₂ alkyl, C₂-C₂₂ alkenyl, and mixtures thereof. R¹ may be branched alkyl and unsaturated alkyl (including polyunsaturated alkyl), wherein the ratio of branched alkyl to unsaturated alkyl is from about 5:95 to about 95:5, preferably from about 75:25 to about 25:75, more preferably from about 50:50 to about 30:70, especially 35:65.

The softener active may also contain alkyl, mono-unsaturated alkylene, and polyunsaturated alkylene groups, with the softener active containing polyunsaturated alkylene groups being at least about 3%, preferably at least about 5%, more preferably at least about 10%, and even more preferably at least about 15%, by weight of the total softener active present. (As used herein, the "percent of softener active" containing a given R¹ group is based upon taking a percentage of the total active based upon the percentage that the given R¹ group is, of the total R¹ groups present.)

The Iodine Value of the "parent fatty acid" of the R¹ group is preferably from about 20 to about 140, more preferably from about 50 to about 130; and most preferably from about 70 to about 115.

For the purposes of the present invention, the R¹ represents the alkyl or alkenyl portion of a fatty acyl source. For example, stearic acid, R¹ CO₂H, comprises R¹ equal to C₁₇ alkyl.

Y units have the formula: 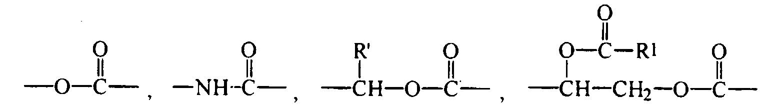 or mixtures thereof; wherein R' is C₁-C₄ alkyl, preferably methyl; preferably Y is 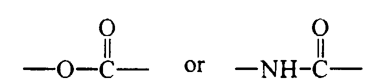 wherein said preferred Y units are taken together with R¹ units to form ester fabric softening actives having the formula: 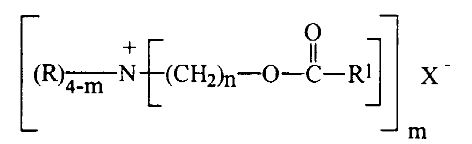 or amide fabric softening actives having the formula: 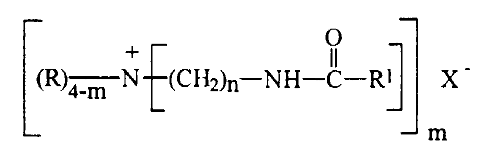 wherein m is an integer from 1 to 3; n is an integer from 1 to 4.

The amines which are used to prepare the fabric softening actives of the present invention have the formula: 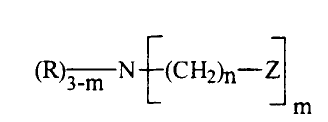 wherein R is the same as defined herein above; each Z is independently selected from the group consisting of -OH, -CHR'OH, -CH(OH)CH₂OH, -NH₂, and mixtures thereof, R' is C₁-C₄ alkyl, preferably methyl; the indices m and n are the same as defined hereinabove.

An example of a preferred type of softener active is a mixture of quaternized amines having the formula: 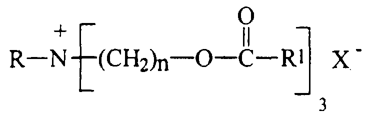 which are derived from the reaction of a triamine with a suitable triglyceride feedstock (a mixture of fatty acids, fatty acid esters, or mixtures thereof) followed by quaternization

An example of a preferred amine starting material for use in Step (b) is the symmetrical triamine, triethanol amine, having the formula: 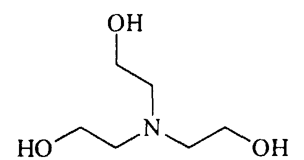 which can be represented by the generic formula: 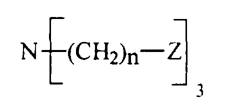 wherein Z is -OH and n is equal to 2. Reaction, preferably a transesterification reaction between fatty acid esters of the triglyceride source and the triamine, yields a softener active precursor mixture having the general formula: 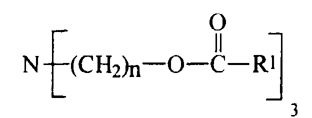 wherein the composition of alkyl units which comprise R¹ is determined by the choice of the triglyceride source, and n is equal to 2. For example, a preferred source of triglyceride is canola oil. Reaction of this triglyceride source, preferably by transesterification to the mixture of canola fatty acid methyl esters, provides a suitable feedstock for reaction with triethanol amine in Step (b) of the present process. Typically, canola oil yields the following R¹CO₂- fatty acyl distribution in the final softener active.

**Table I**

| Fatty acyl unit | % |
|---|---|
| C14 | 0.1 |
| C16 | 5.4 |
| C16:1 | 0.4 |
| C18 | 5.7 |
| C18:1 | 67.0 |
| C18:2 | 13.5 |
| C18:3 | 2.7 |
| C20 | 0.5 |
| C20:1 | 4.6 |

The final step in the preparation of the softener active is reaction with a quaternizing reagent, for example, methyl chloride, to form the final product having the formula: 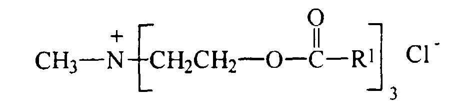 wherein R¹ when taken together with a carboxyl moiety represents the fatty acid distribution outlined above in Table I .

It will be understood by those skilled in the art that the Y unit of the present invention comprises a carboxyl moiety which is introduced into the softener precursor molecules via the source of fatty acyl units. Preferably Y is -O-C(O)- or -NH-C(O)-, however, when the starting amine comprises a branching unit or a di-hydroxyl unit, Y is represented by the formulas: 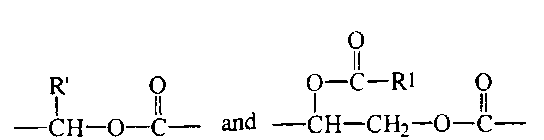 respectively. Starting amines having the formula: 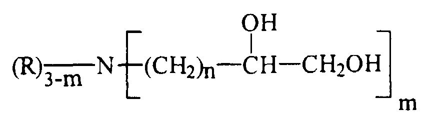 after reaction with a suitable fatty acyl feedstock and quaternization, yield fabric softener actives having the formula: 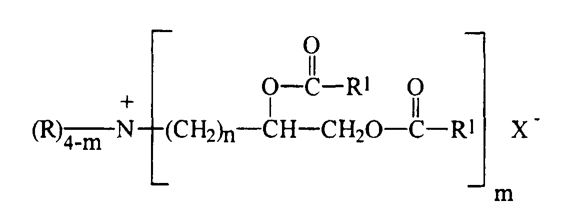 wherein the index m can have the value from 1 to 3, preferably m has the value 1.

For the purposes of the present invention, R moieties which are introduced during the quaternization step are preferably methyl. In the case of amines having the formula: 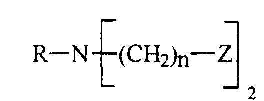 R is preferably the same moiety (i.e. methyl) which is introduced during the quaternization step. For example, a methyl amine having the formula: 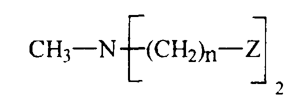 is preferably quaternized to the softener active having the formula:

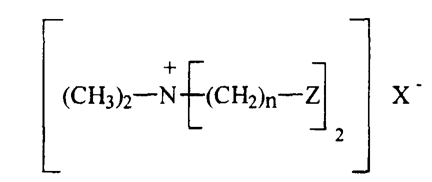

R¹ may be branched alkyl and unsaturated alkyl (including polyunsaturated alkyl), wherein the ratio of branched alkyl to unsaturated alkyl is from about 5:95 to about 95:5, preferably from about 75:25 to about 25:75, more preferably from about 50:50 to about 30:70, especially 35:65.

In one embodiment of the present process, the fabric softening active precursor amine mixture is not full quaternized, that is, some free amine having the general formula: 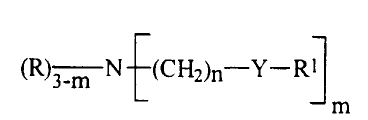 is still present in the final fabric softener mixture or premix.

The softener active may also contain alkyl, mono-unsaturated alkylene, and polyunsaturated alkylene groups, with the softener active containing polyunsaturated alkylene groups being at least about 3%, preferably at least about 5%, more preferably at least about 10%, and even more preferably at least about 15%, by weight of the total softener active present. (As used herein, the "percent of softener active" containing a given R¹ group is based upon taking a percentage of the total active based upon the percentage that the given R¹ group is, of the total R¹ groups present.)

The Iodine Value of the "parent fatty acid" of the R¹ group is preferably from about 20 to about 140, more preferably from about 50 to about 130; and most preferably from about 70 to about 115; and wherein the counterion, X⁻, can be any softener-compatible anion, preferably, chloride, bromide, methylsulfate, ethylsulfate, sulfate, and/or nitrate, more preferably chloride and methylsulfate.

### Diester and/or Diamide Quaternary Ammonium Fabric Softening Active Compound (DEQA)

The preferred fabric softening actives of the present invention are the Diester and/or Diamide Quaternary Ammonium (DEQA) compounds, the diesters having the formula: 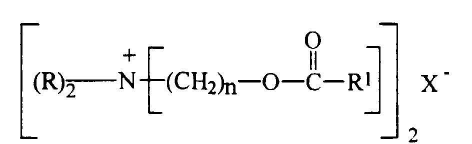 and the diamides having the formula: 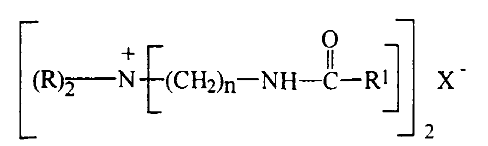 wherein R, R¹, X, and n are the same as defined herein above.

Non-limiting examples of preferred amines which are used to form the DEQA fabric softening actives according to the present invention include methyl bis(2-hydroxyethyl)amine having the formula: 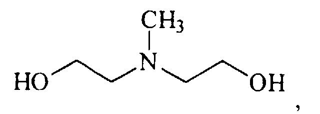 methyl bis(2-hydroxypropyl)amine having the formula: 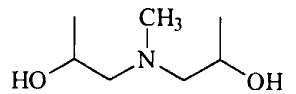 methyl (3-aminopropyl) (2-hydroxyethyl)amine having the formula: 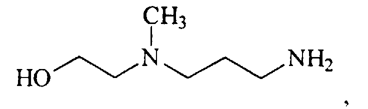 and methyl bis(2-aminoethyl)amine having the formula:

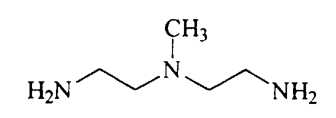

The counterion, X⁽⁻⁾ above, can be any softener-compatible anion, preferably the anion of a strong acid, for example, chloride, bromide, methylsulfate, ethylsulfate, sulfate, nitrate and the like, more preferably chloride. The anion can also, but less preferably, carry a double charge in which case X⁽⁻⁾ represents half a group.

Each source of triglyceride has a different fatty acid composition and therefor the choice of starting triglyceride in Step (a) determines the values for the fatty acyl moiety in the final softener active and therefore the values of R¹. The source of triglyceride can be vegetable oils or animal fats. Preferred vegetable oils include *inter alia* hydrogenated, partially hydrogenated, or non-hydrogenated canola oil, safflower oil, peanut oil, sunflower oil, corn oil, soybean oil, tall oil, and rice bran oil. Tallow is a preferred source of triglycerides derived from animals.

The R¹ units are typically mixtures of linear and branched chains of both saturated and unsaturated aliphatic fatty acids, an example of which (canola oil), is described hereinabove in Table I. The formulator, depending upon the desired physical and performance properties of the final fabric softener active, can choose any of the above mentioned sources of fatty acyl moieties, or alternatively, the formulator can mix sources of triglyceride to form a "customized blend". However, those skilled in the art of fats and oils recognize that the fatty acyl composition may vary, as in the case of vegetable oil, from crop to crop, or from variety of vegetable oil source to variety of vegetable oil source.

DEQA's which are prepared using fatty acids derived from natural sources are preferred. Parameters which favor DEQA performance are *inter alia* varying chain length of the fatty acyl moiety, presence of fatty acyl moieties which have at least one degree of unsaturation (e.g. oleic acid), and when present, sites of unsaturation (primarily double bonds) are predominately in the more desired *cis* configuration. For the purposes of the present invention the term "mixed branched-chain fatty acyl units" is defined as "a mixture of fatty acyl units comprising alkyl and alkenyl chains having from 10 carbons to 22 carbon atoms including the carbonyl carbon atom, and in the case of alkenyl chains, from one to three double bonds, preferably all double bonds in the *cis* configuration". It is preferred that at least a substantial percentage of the fatty acyl groups are unsaturated, e.g., from about 25%, preferably from about 50% to about 70%, preferably to about 65%. The total level of fabric softening active containing polyunsaturated fatty acyl groups (TPU) can be from about 3%, preferably from about 5%, more preferably from about 10% to about 30%, preferably to about 25%, more preferably to about 18%. As stated herein above *cis* and *trans* isomers can be used, preferably with a *cis/trans* ratio of from 1:1, preferably at least 3:1, and more preferably from about 4:1 to about 50:1, more preferably about 20:1, however, the minimum being 1:1.

The mixed branched-chain fatty acyl materials are easier to formulate than conventional saturated branched chain fabric softener actives and especially actives derived from only one type of acyl moiety (i.e. all stearoyl units). They can be used to form concentrated premixes that maintain their low viscosity and are therefore easier to process, e.g., pump, mix, etc. These materials with only the low amount of solvent that normally is associated with such materials, i.e., from about 5%, preferably from about 8%, more preferably from about 10% to about 20%, preferably to about 25%, more preferably to about 20%, weight of the total softener/solvent mixture, are also easier to formulate into concentrated, stable compositions of the present invention, even at ambient temperatures. This ability to process the actives at low temperatures is especially important for the polyunsaturated groups, since it minimizes degradation. Additional protection against degradation can be provided when the compounds and softener compositions contain effective antioxidants and/or reducing agents, as disclosed hereinafter. The use of branched chain fatty acyl groups improves the resistance to degradation while maintaining fluidity and improving softening.

### Water soluble organic solvent system

Stable compositions optionally comprise from about 5%, preferably from about 8%, more preferably from about 10% to about 30%, preferably to about 25%, more preferably to about 20%, by weight of the composition of water soluble organic solvent. The solvent is preferably mixed with the fabric softener DEQA to help provide a low viscosity for ease of processing, e.g., pumping and/or mixing, even at ambient temperatures.

The organic solvent is preferably water soluble solvent, e.g., ethanol; isopropanol; 1,2-propanediol; 1,3-propanediol; propylene carbonate, hexylene gylcol; etc.

The ability to create finished concentrated compositions with conventional mixing at ambient temperatures, e.g., from about 10°C, preferably from about 20°C to about 40°C, preferably to about 35°C, with only low levels of water soluble solvents, is possible with the fabric softener compounds disclosed hereinbefore. This processing at ambient temperatures is very important when the dispersion compositions contain high levels of polyunsaturated softener active materials.

### Stabilizers

Stabilizers are highly desirable in finished compositions. The term "stabilizer," as used herein, includes antioxidants and reductive agents. These agents are present at a level of from 0%, preferably from about 0.001%, more preferably from about 0.01%, even more preferably from about 0.035% to about 2.0%, preferably to about 0.2%, more preferably to about 0.1% for antioxidants, and more preferably from about 0.01% to about 0.2% for reductive agents, in either the formed softener active or in the final composition. For the premix, the levels are adjusted, depending on the concentrations of the softener active in the premix and the finished composition. These assure good odor stability under long term storage conditions. Antioxidants and reductive agent stabilizers are especially critical for unscented or low scent products (no or low perfume).

Examples of antioxidants that can be added to the dispersion compositions include a mixture of ascorbic acid, ascorbic palmitate, propyl gallate, available from Eastman Chemical Products, Inc., under the trade names Tenox® PG and Tenox® S-1; a mixture of BHT (butylated hydroxytoluene), BHA (butylated hydroxyanisole), propyl gallate, and citric acid, available from Eastman Chemical Products, Inc., under the trade name Tenox®-6; butylated hydroxytoluene, available from UOP Process Division under the trade name Sustane® BHT; tertiary butylhydroquinone, Eastman Chemical Products, Inc., as Tenox® TBHQ; natural tocopherols, Eastman Chemical Products, Inc., as Tenox® GT-1/GT-2; and butylated hydroxyanisole, Eastman Chemical Products, Inc., as BHA; long chain esters (C₈-C₂₂) of gallic acid, e.g., dodecyl gallate; Irganox® 1010; Irganox® 1035; Irganox® B 1171; Irganox® 1425; Irganox® 3114; Irganox® 3125; and mixtures thereof; preferably Irganox® 3125, Irganox® 1425, Irganox® 3114, and mixtures thereof; more preferably Irganox® 3125 alone or mixed with citric acid and/or other chelators such as isopropyl citrate, Dequest® 2010, available from Monsanto with a chemical name of 1-hydroxyethylidene-1,1-diphosphonic acid (etidronic acid), and Tiron®, available from Kodak with a chemical name of 4,5-dihydroxy-m-benzene-sulfonic acid/sodium salt.

### Water Soluble Salts

Particularly preferred ingredients include water soluble calcium and/or magnesium compounds, which provide additional stability. The chloride salts are preferred, but acetate, nitrate, etc. salts can be used. The level of calcium and/or magnesium salts is from 0% to about 2%, preferably from about 0.05% to about 0.5%, more preferably from about 0.1% to about 0.25%. These materials are desirably added to the water and/or acid (water seat) used to prepare the finished dispersion compositions to help adjust the finished viscosity.

### Chelating Agents

The compositions formed via the present invention may include one or more chelating agents such as copper and/or nickel chelating agents ("chelators") apart from the DTPA or EDDS which is added during the formation of the fabric softening active and the fabric softening composition. The chelating agent may be present in the composition in the range of from 0.001% to 10% by weight of the composition. More preferably the chelant is present in the range of from about 0.01% to about 5% and most preferably in the range of from about 0.01% to about 3% by weight of the composition.

Such water-soluble chelating agents can be selected from the group consisting of amino carboxylates, amino phosphonates, polyfunctionally-substituted aromatic chelating agents and mixtures thereof, all as hereinafter defined and all preferably in their acidic form. Amino carboxylates useful as chelating agents herein include ethylenediaminetetraacetic acid (EDTA), N-hydroxyethylethylenediaminetriacetates, nitrilotriacetates (NTA), ethylenediamine tetraproprionates, ethylenediamine-N,N'-diglutamates, 2-hyroxypropylenediamine-N,N'-disuccinates, triethylenetetraaminehexacetates, diethylenetriaminepentaacetates (DETPA) and ethanoldiglycines, including their water-soluble salts such as the alkali metal, ammonium, and substituted ammonium salts thereof and mixtures thereof.

Amino phosphonates are also suitable for use as chelating agents in the compositions of the invention when at least low levels of total phosphorus are permitted in detergent compositions, and include ethylenediaminetetrakis (methylenephosphonates), diethylenetriamine-N,N,N',N",N"-pentakis(methane phosphonate) (DETMP) and 1-hydroxyethane-1,1-diphosphonate (HEDP). Preferably, these amino phosphonates to not contain alkyl or alkenyl groups with more than about 6 carbon atoms.

The chelating agents are typically used in the present rinse process at levels from about 2 ppm to about 25 ppm, for periods from 1 minute up to several hours' soaking.

As can be seen from the foregoing, a wide variety of chelators may be added to the compositions. Indeed, simple polycarboxylates such as citrate, oxydisuccinate, and the like, may also be used, although such chelators are not as effective as the amino carboxylates and phosphonates, on a weight basis. Accordingly, usage levels may be adjusted to take into account differing degrees of chelating effectiveness. The chelators herein will preferably have a stability constant (of the fully ionized chelator) for copper ions of at least about 5, preferably at least about 7. Typically, the chelators will comprise from about 0.5% to about 10%, more preferably from about 0.75% to about 5%, by weight of the compositions herein. Preferred chelators include DETMP, DETPA, NTA, EDTA and mixtures thereof.

### Principal Solvent System

The compositions of the present invention may comprise a principal solvent system. This is particularly the case when formulating liquid, clear fabric softening compositions. When employed, the principal solvent preferably comprises less than about 40%, preferably from about 10% to about 35%, more preferably from about 12% to about 25%, and even more preferably from about 14% to about 20%, by weight of the composition. The principal solvent is selected to minimize solvent odor impact in the composition and to provide a low viscosity to the final composition. For example, isopropyl alcohol is not very effective and has a strong odor. n-Propyl alcohol is more effective, but also has a distinct odor. Several butyl alcohols also have odors but can be used for effective clarity/stability, especially when used as part of a principal solvent system to minimize their odor. The alcohols are also selected for optimum low temperature stability, that is they are able to form compositions that are liquid with acceptable low viscosities and translucent, preferably clear, down to about 40°F (about 4.4°C) and are able to recover after storage down to about 20°F (about 6.7°C).

The suitability of any principal solvent for the formulation of the liquid, concentrated, preferably clear, fabric softener compositions herein with the requisite stability is surprisingly selective. Suitable solvents can be selected based upon their octanol/water partition coefficient (P). Octanol/water partition coefficient of a principal solvent is the ratio between its equilibrium concentration in octanol and in water. The partition coefficients of the principal solvent ingredients of this invention are conveniently given in the form of their logarithm to the base 10, logP.

The logP of many ingredients has been reported; for example, the Pomona92 database, available from Daylight Chemical Information Systems, Inc. (Daylight CIS), Irvine, California, contains many, along with citations to the original literature. However, the logP values are most conveniently calculated by the "CLOGP" program, also available from Daylight CIS. This program also lists experimental logP values when they are available in the Pomona92 database. The "calculated logP" (ClogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990, incorporated herein by reference). The fragment approach is based on the chemical structure of each ingredient, and takes into account the numbers and types of atoms, the atom connectivity, and chemical bonding. These ClogP values, which are the most reliable and widely used estimates for this physicochemical property, are preferably used instead of the experimental logP values in the selection of the principal solvent ingredients which are useful in the present invention. Other methods that can be used to compute ClogP include, e.g., Crippen's fragmentation method as disclosed in J. Chem. Inf. Comput. Sci., 27, 21 (1987); Viswanadhan's fragmentation method as disclose in J. Chem. Inf. Comput. Sci., 29, 163 (1989); and Broto's method as disclosed in Eur. J. Med. Chem. - Chim. Theor., 19, 71 (1984). The principal solvents herein are selected from those having a ClogP of from about 0.15 to about 0.64, preferably from about 0.25 to about 0.62, and more preferably from about 0.40 to about 0.60, said principal solvent preferably being at least somewhat asymmetric, and preferably having a melting, or solidification, point that allows it to be liquid at, or near room temperature. Solvents that have a low molecular weight and are biodegradable are also desirable for some purposes. The more assymetric solvents appear to be very desirable, whereas the highly symmetrical solvents such as 1,7-heptanediol, or 1,4-bis(hydroxymethyl) cyclohexane, which have a center of symmetry, appear to be unable to provide the essential clear compositions when used alone, even though their ClogP values fall in the preferred range.

The most preferred principal solvents can be identified by the appearance of the softener vesicles, as observed via cryogenic electron microscopy of the compositions that have been diluted to the concentration used in the rinse. These dilute compositions appear to have dispersions of fabric softener that exhibit a more unilamellar appearance than conventional fabric softener compositions. The closer to uni-lamellar the appearance, the better the compositions seem to perform. These compositions provide surprisingly good fabric softening as compared to similar compositions prepared in the conventional way with the same fabric softener active.

Operable principal solvents are disclosed and listed below which have ClogP values which fall within the requisite range. These include mono-ols, C6 diols, C7 diols, octanediol isomers, butanediol derivatives, trimethylpentanediol isomers, ethylmethylpentanediol isomers, propyl pentanediol isomers, dimethylhexanediol isomers, ethylhexanediol isomers, methylheptanediol isomers, octanediol isomers, nonanediol isomers, alkyl glyceryl ethers, di(hydroxy alkyl) ethers, and aryl glyceryl ethers, aromatic glyceryl ethers, alicyclic diols and derivatives, C₃C₇ diol alkoxylated derivatives, aromatic diols, and unsaturated diols. Particularly preferred principal solvents include hexanediols such as 1,2-Hexanediol and 2-Ethyl-1,3-hexanediol and pentanediols such as 2,2,4-Trimethyl-1,3-pentanediol. These principal solvents are all disclosed in copending U.S. Patent application numbers 08/621,019; 08/620,627; 08/620,767; 08/620,513; 08/621,285; 08/621,299; 08/621,298; 08/620,626; 08/620,625; 08/620,772; 08/621,281; 08/620,514; and 08/620,958, all filed March 22, 1996 and all having the title "CONCENTRATED, STABLE, PREFERABLY CLEAR, FABRIC SOFTENING COMPOSITION", the disclosures of which are all herein incorporated by reference.

### Optional Ingredients

Other optional ingredients useful in compositions of the present invention include, but are not limited to, dye transfer inhibiting agents, polymeric dispersing agents, soil release agents, scum dispersants, suds suppressors, optical brighteners or other brightening or whitening agents, dye fixing agents, light fading protection agents, oxygen bleach protection agents, fabric softening clay, anti-static agents, other active ingredients, carriers, hydrotropes, processing aids, dyes or pigments, bactericides, colorants, perfumes, preservatives, opacifiers, anti-shrinkage agents, anti-wrinkle agents, fabric crisping agents, spotting agents, germicides, fungicides, anti-corrosion agents, and the like.

The synthesis of the mixtures of biodegradable fabric softening actives of the present invention is further illustrated in the following Synthesis Examples. These Synthesis Examples are provided for purposes of illustration only.

### Compound Synthesis Example A

Approximately 1,300 grams of food grade (refined, bleached, degummed) canola oil and approximately 6.5 grams of a commercial nickel hydrogenation catalyst (Engelhard, "N-545"®) corresponding to approximately 0.13 wt.% Ni, are placed in a hydrogenation reactor which is equipped with stirrer. 1000 ppm of chelant may be added at this point, however, the activity of the catalyst may be affected. The reactor is sealed and evacuated. The contents are heated to about 170°C and hydrogen is fed into the reactor. Stirring at 450 rpm is maintained throughout the reaction. After about 10 minutes the temperature in the reactor is about 191°C and the hydrogen pressure is about 11 psig. The temperature is held at about 190°C. After about 127 minutes from when the hydrogen feed began, the hydrogen pressure is about 10 psig. A sample of the reaction mass is drawn and found to have an Iodine Value of about 78.0 and a *cis:trans* ratio of about 1.098. After another about 20 minutes at about 190°C, the hydrogen pressure is about 9.8 psig. The hydrogen feed is discontinued and the reactor contents cooled with stirring. The chelant may be added at this point if the chelant tends to affect the activity of the hydrogenation catalyst. In addition, any anti-oxidant may be added at this point. The final reaction product has an Iodine Value of about 74.5 and a cis:trans ratio of about 1.35.

The product that forms in the reactor is removed and filtered. It has a cloud point of about 22.2°C. The chain length distributions of the acyl substituents on the sample taken at about 127 minutes, and of the final product, are determined to be as shown in Table 1 in which "sat." means saturated, and "mono", "di" and "tri" means mono-unsaturated, di-unsaturated, and tri-unsaturated respectively.

**TABLE 1**

| Approximate Percent (mol.) | | |
|---|---|---|
| Chain length | Sample @ 127 min. | Product |
| C14-sat. | 0.1 | 0.1 |
| C16-sat. | 4.7 | 4.6 |
| C16-mono. | 0.4 | 0.4 |
| C18-sat. | 8.9 | 13.25 |
| C18-mono. | 77.0 | 73.8 |
| C18-di. | 4.5 | 3.1 |
| C20-sat. | 0.7 | 0.75 |
| C-20-mono. | 2.1 | 2.0 |
| Other | 1.6 | 2.0 |

### Compound Synthesis Example B

About 1,300 grams of food grade canola oil and about 5.2 grams of Engelhard "N-545"® nickel hydrogenation catalyst are placed in a hydrogenation reactor which is equipped with a stirrer. The reactor is sealed and evacuated. The contents are heated to about 175°C and hydrogen is fed into the reactor. Stirring is maintained at about 450 rpm throughout the course of reaction. After about 5 minutes the temperature in the reactor is about 190°C and the hydrogen pressure is about 7 psig. The temperature is held at about 190°C. After about 125 minutes from the start of the hydrogen feed, the hydrogen pressure is about 7 psig. A sample of the reaction mass is drawn and found to have an Iodine Value of 85.4. After another about 20 minutes at about 190°C, the hydrogen pressure is about 6 psig. The hydrogen feed is discontinued, the mixture is treated with 950 ppm of DTPA and optionally an anti-oxidant, and the reactor contents cooled with stirring. The final reaction product has an Iodine Value of about 80.0. The product that forms in the reactor is removed and filtered. It has a cloud point of about 18.6°C.

### Compound Synthesis Example C

About 1,300 grams of food grade canola oil and about 2.9 grams of Engelhard "N-545"® nickel hydrogenation catalyst are placed in a hydrogenation reactor which is equipped with a stirrer. The reactor is sealed and evacuated. The contents are heated to about 180°C and hydrogen is fed into the reactor. Stirring is maintained at about 450 rpm throughout the course of the reaction. After about 5 minutes the temperature in the reactor is about 192°C and the hydrogen pressure is about 10 psig. The temperature is held at about 190 ±3°C. After about 105 minutes from the start of the hydrogen feed, the hydrogen pressure is about 10 psig. A sample of the reaction mass is drawn and found to have an Iodine Value of 85.5. After another about 20 minutes at about 190°C, the hydrogen pressure is about 10 psig. The hydrogen feed is discontinued, the mixture is treated with 950 ppm of DTPA and optionally an anti-oxidant, and the reactor contents cooled with stirring. The final reaction product has an Iodine Value of about 82.4. The product that forms in the reactor is removed and filtered. It has a cloud point of about 17.2°C.

### Compound Synthesis Example D

About 1,300 grams of food grade canola oil and about 1.4 grams of Engelhard "N-545"® nickel hydrogenation catalyst are placed in a hydrogenation reactor which is equipped with a stirrer. The reactor is sealed and evacuated. The contents are heated to about 180°C and hydrogen is fed into the reactor. After 5 minutes the temperature in the reactor is about 191°C and the hydrogen pressure is about 10 psig. The temperature is held at about 190 ±3°C. After about 100 minutes from the start of the hydrogen feed, the hydrogen pressure is about 10 psig. A sample of the reaction mass is drawn and found to have an Iodine Value of about 95.4. After another about 20 minutes at about 190°C, the hydrogen pressure is about 10 psig. The hydrogen feed is discontinued, the mixture is treated with 950 ppm of DTPA and optionally an anti-oxidant, and the reactor contents cooled with stirring. The final reaction product had an Iodine Value of about 2.3. The product that forms in the reactor is removed and filtered. It has a cloud point of about 34°C.

### Compound Synthesis Example E

About 1,300 grams of food grade canola oil and about 1.3 grams of Engelhard "N-545"® nickel hydrogenation catalyst are placed in a hydrogenation reactor which is equipped with a stirrer. The reactor is sealed and evacuated. The contents are heated to about 190°C and hydrogen is fed into the reactor to a hydrogen pressure of about 5 psig. After about 3 hours from the start of the hydrogen feed, a sample of the reaction mass is drawn and found to have an iodine value of about 98. The hydrogenation is interrupted, another about 0.7 grams of the same catalyst is added, and the reaction conditions are reestablished at about 190°C for another about 1 hour. The hydrogen feed is then discontinued, the mixture is treated with 950 ppm of DTPA and optionally an anti-oxidant, and the reactor contents cooled with stirring. The final reaction product had an Iodine Value of about 89.9. The product that forms in the reactor is removed and filtered. It has a cloud point of about 16.0°C.

### Compound Synthesis Example F

About 1,300 grams of food grade canola oil and about 2.0 grams of Engelhard "N-545"® nickel hydrogenation catalyst are placed in a hydrogenation reactor which is equipped with a stirrer. The reactor is sealed and evacuated. The contents are heated to about 190°C and hydrogen is fed into the reactor to a hydrogen pressure of about 5 psig. Stirring is maintained at about 420 rpm throughout the course of reaction of the hydrogen feed. After about 130 minutes from the start of the hydrogen feed, the hydrogen feed is discontinued, the mixture is treated with 900 ppm of DTPA and optionally an anti-oxidant, and the reactor contents cooled with stirring. The final reaction product had an Iodine Value of about 96.4. The product that forms in the reactor is removed and filtered. It has a cloud point of about 11.2°C.

### Compound Synthesis Example G

A mixture of about 1,200 grams of the hydrogenated oil from Synthesis Example F and about 200 grams of the hydrogenated oil from Synthesis Example A is hydrolyzed three times in the presence of 900 ppm DTPA with about 250°C steam at about 600 psig for about 2.5 hours at a ratio of steam:oil of about 1.2 (by weight). An aqueous solution containing the glycerine which had split off is removed.

The resulting mixture of fatty acids is vacuum distilled for a total of about 150 minutes, in which the pot temperature rose gradually from about 200°C to about 238°C and the head temperature rose gradually from about 175°C to about 197°C. Vacuum of about 0.3-0.6 mm is maintained.

The fatty acids product of the vacuum distillation has an Iodine Value of about 99.1, an amine value (AV) of about 197.6 and a saponification value (SAP) of about 198.6.

### Compound Synthesis Example H

About 800 grams of mixture of fatty acids obtained from canola oil by the procedure of Example G, about 194.4 grams of MDEA (methyl diethanolamine) about 2 grams of BHT (butylated hydroxytoluene), 1000 ppm of DTPA, and about 1 gram of an approximately 50 wt.% aqueous solution of HPPA are placed in a pot at the bottom of a distillation column. Nitrogen flow through the column is established. The pot is heated, and distillation began at a pot temperature of about 150°C, and a head temperature of about 102°C. The mixture temperature rose to about 193°C in the first hour and then gradually rose to about 202°C through the next about 4 hours. The head temperature rose to about 107°C in the first hour and then declined gradually to about 62°C over the next about 4 hours. The product in the pot is then cooled, recovered and analyzed. The distillate contained about 3 wt.% MDEA, about 51 grams of water, and exhibited a total amine value (TAV) of about 0.5. The product remaining in the pot has a total amine value (TAV) of about 93.3.

### Compound Synthesis Example I

About 900 grams of the product of Synthesis Example H, about 158 grams of ethanol, approximately 1200 ppm of DTPA neutralized to a pH of about 6.8, about 0.15 grams of antifoam, and sufficient methyl chloride to establish an initial pressure of about 43 psig are combined in a sealed reactor. After about 7 minutes the temperature is about 106°C and the pressure is about 84 psig. The contents are then maintained at about 105±1°C for about 3-5 hours while the pressure is maintained at about 57±2 psig by additions of methyl chloride. Then the reactor is vented, and the contents cooled to about 95°C. A total of about 110 grams of methyl chloride is used. The product is then removed and stripped at about 65°C on a rotary evaporator. Approximately 350 ppm of Irganox® 3125 antioxidant is added and mixed in. The product has a diester content of about 75.9%, a monoester content of about 11.4%, approximately 980 ppm of DTPA and is nearly clear in color.

### Compound Synthesis Example J

The synthesis of Examples G, H, and I are repeated employing 1200 grams of non-hydrogenated canola oil in Example G. The product has approximately 980 ppm of DTPA and is nearly clear in color.

The following are suitable N,N-di(unsaturated fatty acyl-oxyethyl)-N,N-dimethyl ammonium chloride fabric softening actives (DEQA's), prepared according to the process of the present invention with approximate distributions of fatty acyl groups given, that are used hereinafter for preparing the following compositions.

| R¹CO₂- * | DEQA¹ | DEQA² | DEQA³ | DEQA⁴ | DEQA⁵ |
|---|---|---|---|---|---|
| C12 | trace | trace | 0 | 0 | 0 |
| C14 | 3 | 3 | 0 | 0 | 0 |
| C14:1 | 3 | 3 | 0 | 0 | 0 |
| C16 | 4 | 4 | 5 | 5 | 5 |
| C16:1 | 11 | 7 | 0 | 0 | 3 |
| C18 | 0 | 0 | 5 | 6 | 6 |
| C18:1 | 74 | 73 | 71 | 68 | 67 |
| C18:2 | 4 | 8 | 8 | 11 | 11 |
| C18:3 | 0 | 1 | 1 | 2 | 2 |
| C20 | 0 | 0 | 2 | 0 | 0 |
| C20:1 | 0 | 0 | 2 | 2 | 2 |
| Unknown | balance | balance | balance | balance | balance |
| Iodine Value | 86-90 | 88-95 | 99 | 100 | 95 |
| *cis/trans* | 20-30 | 20-30 | 4 | 5 | 5 |
| TPU** | 4 | 9 | 10 | 13 | 13 |

| | | | | | |
|---|---|---|---|---|---|
| * Composition of the fatty acyl group derived from the triglyceride source. | | | | | |
| ** Total Polyunsaturated fatty acyl groups, by weight. | | | | | |

| R¹CO₂- * | DEQA⁶ | DEQA⁷ | DEQA⁸ | DEQA⁹ |
|---|---|---|---|---|
| C14 | 0 | 1 | 0 | 0 |
| C14:1 | 0 | 0 | 0 | 0 |
| C16 | 11 | 25 | 5 | 4 |
| C16:1 | 1 | 0 | 1 | 0 |
| C18 | 4 | 20 | 14 | 2 |
| C18:1 | 27 | 45 | 74 | 73 |
| C18:2 | 50 | 6 | 3 | 11 |
| C18:3 | 7 | 0 | 0 | 3 |
| Other | balance | balance | balance | balance |
| Iodine Value | 125-138 | 56 | N/A | N/A |
| *cis/trans* | N/A | 7 | N/A | 3 |
| TPU** | 57 | 6 | N/A | 14 |

| | | | | |
|---|---|---|---|---|
| * Composition of the fatty acyl group derived from the triglyceride source. | | | | |
| ** Total Polyunsaturated fatty acyl groups, by weight. N/A = not available. | | | | |

The compositions in the Examples below are made by first preparing a softener premix by blending at room temperature the appropriate DEQA or mixture of DEQA actives. The softener actives can be heated to melting at, e.g., about 130-150°F (about 55-66°C), if the softener active(s) is not fluid at room temperature. The softener active is mixed using an IKA RW 25® mixer for about 2 to about 5 minutes at about 150 rpm. Separately, an acid/water seat is prepared by mixing the HCl with deionized (DI) water at room temperature. If the softener actives and/or the principal solvent(s) are not fluid at room temperature and need to be heated, the acid/water seat should also be heated to about 100°F (about 38°C) and maintaining said temperature with a water bath. The principal solvent(s) (melted at suitable temperatures if their melting points are above room temperature) are added to the softener premix and said premix is mixed for about 5 minutes. The acid/water seat is then added to the softener premix and mixed for about 20 to about 30 minutes or until the composition is clear and homogeneous. The composition is allowed to air cool to ambient temperature, if necessary.

### EXAMPLES 1-9

**TABLE I**

| | weight % | | |
|---|---|---|---|
| Ingredients | **1** | **2** | **3** |
| DEQA² (85% active in ethanol) | 19.9 | -- | 15.3 |
| DEQA⁸ (85% active in ethanol) | -- | 19.9 | -- |
| DEQA⁹ (85% active in ethanol) | 10.7 | 10.7 | 15.3 |
| Ethanol | -- | -- | 2 |
| Irganox® 3125 | 0.01 | 0.01 | 0.02 |
| DTPA | 0.01 | 0.01 | 0.01 |
| 1,2-Hexanediol | 18 | 18 | 18 |
| Perfume | 1.2 | 1.2 | 1 |
| HCl (pH 2.5-4.0) | 0.005 | 0.005 | 0.005 |
| Distilled Water | balance | balance | balance |

**TABLE II**

| | weight % | | |
|---|---|---|---|
| Ingredients | **4** | **5** | **6** |
| DEQA² (85% active in ethanol) | -- | 32.5 | -- |
| DEQA⁸ (85% active in ethanol) | 15.3 | -- | 32.5 |
| DEQA⁹ (85% active in ethanol) | 15.3 | 17.5 | 17.5 |
| Ethanol | 2 | 2 | 2 |
| Irganox® 3125 | 0.02 | 0.03 | 0.03 |
| DTPA | 0.1 | 0.1 | 0.1 |
| 1,2-Hexanediol | 18 | 28 | 28 |
| Perfume | 1.35 | 1.3 | 1.3 |
| HCl (pH 2.5-4.0) | 0.005 | 0.005 | 0.005 |
| Distilled Water | balance | balance | balance |

**TABLE III**

| | weight % | | |
|---|---|---|---|
| Ingredients | **7** | **8** | **9** |
| DEQA⁸ (85% active in ethanol/hexylene glycol | 30.6 | 30.6 | -- |
| DEQA¹⁰ (85% active in ethanol/hexylene glycol 1:1) | -- | -- | 30.6 |
| 2,2,4-Trimethylpentane-1,3-diol | 15.0 | -- | 12.0 |
| Irganox® 3125 | 0.01 | 0.01 | 0.01 |
| DTPA | 0.01 | 0.01 | 0.01 |
| 2-Ethyl-1,3-hexanediol | -- | 15.0 | -- |
| 1,4-Cyclohexanedimethanol | 5.0 | 5.0 | 5.0 |
| Perfume | 2.5 | 0.5 | 1.5 |
| HCl (pH 2.5-4.0) | 0.005 | 0.005 | 0.005 |
| Distilled Water | balance | balance | balance |

## Claims

1. A process for preparing a fabric softening active comprising the steps of:
a) providing a source of triglyceride and reacting said source of triglyceride to form a mixture, said mixture comprising fatty acids having the formula R¹CO₂H, fatty acid esters having the formula R¹CO₂R", or mixtures thereof; wherein R¹ is C₁-C₂₂ alkyl, C₂-C₂₂ alkenyl, and mixtures thereof; R" is C₁-C₄ alkyl, C₂-C₄ monohydroxyalkyl, C₃-C₄ dihydroxyalkyl;
b) reacting said mixture from step (a) with one or more amines having the formula: 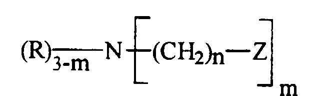 wherein R is C₁-C₆ alkyl, C₁-C₆ hydroxyalkyl, benzyl, or mixtures thereof; each Z is independently selected from the group consisting of -OH, -CHR'OH, -CH(OH)CH₂OH, -NH₂, and mixtures thereof, wherein R' is C₁-C₄ alkyl; m is an integer from 1 to 3; n is an integer from 1 to 4; to form a fabric softening active precursor mixture having the formula: 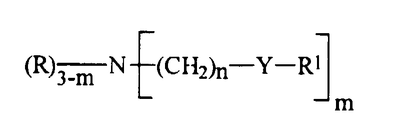 wherein R, R¹, m, and n are the same as above; Y has the formula: 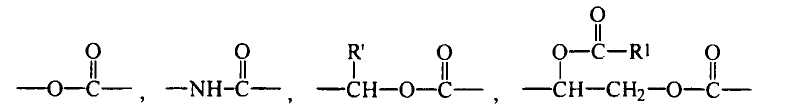 or mixtures thereof; wherein R¹ and R' are the same as above; and
c) quaternizing said fabric softening active precursor mixture from step (b) by reacting said mixture with a quaternizing agent of the formula RX, wherein R is the same as defined in step (b) and X is a softener compatible anion, forming a fabric softening active having the formula: 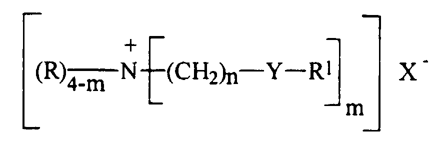
wherein R, R¹, X, Y, m, and n are the same as above; provided at least step (c) is carried out in the presence of a chelating agent selected from the group consisting of diethylenetriaminepentaacetic acid, ethylenediamine-N,N'-disuccinnic acid, and mixtures thereof.

2. The process as claimed in Claim 1 wherein said step of reacting said source of triglyceride further includes reacting in the presence of said chelating agent and said step of esterifying further includes esterifying in the presence of said chelating agent.

3. The process as claimed in either Claim 1 or 2 wherein the total amount of chelating agent added to said process is within the range of from 10 ppm to 5,000 ppm by weight, of formed active.

4. The process as claimed in any of Claims 1-3 said source of triglyceride is selected from the group consisting of tallow, partially hydrogenated tallow, lard, partially hydrogenated lard, vegetable oils, partially hydrogenated vegetable oils, and mixtures thereof.

5. A process according to any of Claims 1-4 wherein said vegetable oil or partially hydrogenated vegetable oil is selected from the group consisting of canola oil, partially hydrogenated canola oil, safflower oil, partially hydrogenated safflower oil, peanut oil, partially hydrogenated peanut oil, sunflower oil, partially hydrogenated sunflower oil, corn oil, partially hydrogenated corn oil, soybean oil, partially hydrogenated soybean oil, tall oil, partially hydrogenated tall oil, rice bran oil, partially hydrogenated rice bran oil, and mixtures thereof.

6. A process according to Claim 1 wherein said process further comprises the step of adding from 0.001% to 2% by weight, of formed active, of an antioxidant compound to any or all of steps (a), (b), or (c)

7. A process for preparing a fabric softening premix composition comprising the steps of:
a) providing a source of triglyceride and reacting said source of triglyceride to form a mixture, said mixture comprising fatty acids having the formula R¹CO₂H, fatty acid esters having the formula R¹CO₂R", or mixtures thereof; wherein R¹ is C₁-C₂₂ alkyl, C₂-C₂₂ alkenyl, and mixtures thereof; R" is C₁-C₄ alkyl, C₂-C₄ monohydroxyalkyl, C₃-C₄ dihydroxyalkyl;
b) reacting said mixture from step (a) with one or more amines having the formula: 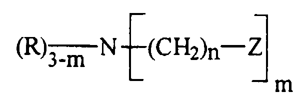 wherein R is C₁-C₆ alkyl, C₁-C₆ hydroxyalkyl, benzyl, or mixtures thereof; each Z is independently selected from the group consisting of -OH, -CHR'OH, -CH(OH)CH₂OH, -NH₂, and mixtures thereof, wherein R' is C₁-C₄ alkyl; m is an integer from 1 to 3; n is an integer from 1 to 4; to form a fabric softening active precursor mixture having the formula: 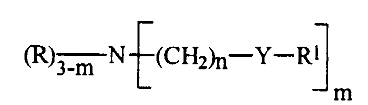 wherein R, R¹, m, and n are the same as above; Y has the formula: 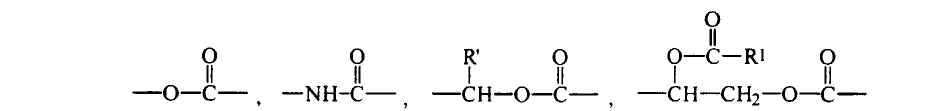 or mixtures thereof; wherein R¹ and R' are the same as above;
c) quaternizing said fabric softening active precursor mixture from step (b) by reacting said mixture with a quaternizing agent of the formula RX, wherein R is the same as defined in step (b) and X is a softener compatible anion, forming a fabric softening active having the formula: 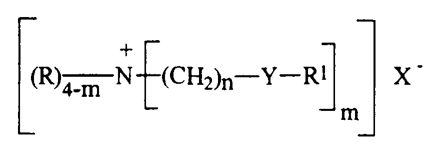 wherein R, R¹, X, Y, m, and n are the same as above; and
d) mixing said fabric softening active with a principal solvent having a ClogP of from 0.15 to 0.64 thereby forming a fabric softener premix;
provided at least step (c) is carried out in the presence of a chelating agent selected from the group consisting of diethylenetriaminepentaacetic acid, ethylenediamine-N,N'-disuccinnic acid, and mixtures thereof.

8. A process for preparing a fabric softening composition comprising the steps of:
a) providing a source of triglyceride and reacting said source of triglyceride to form a mixture, said mixture comprising fatty acids having the formula R¹CO₂H, fatty acid esters having the formula R¹CO₂R", or mixtures thereof; wherein R¹ is C₁-C₂₂ alkyl, C₂-C₂₂ alkenyl, and mixtures thereof; R" is C₁-C₄ alkyl, C₂-C₄ monohydroxyalkyl, C₃-C₄ dihydroxyalkyl;
b) optionally, hydrogenating said mixture formed in step (a);
c) reacting said mixture from step (a) with one or more amines having the formula: 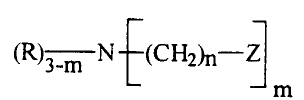 wherein R is C₁-C₆ alkyl, C₁-C₆ hydroxyalkyl, benzyl, or mixtures thereof; each Z is independently selected from the group consisting of -OH, -CHR'OH, -CH(OH)CH₂OH, -NH₂, and mixtures thereof, wherein R' is C₁-C₄ alkyl; m is an integer from 1 to 3; n is an integer from 1 to 4; to form a fabric softening active precursor mixture having the formula: 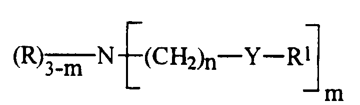 wherein R, R¹, m, and n are the same as above; Y has the formula: 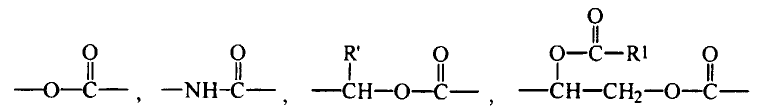 or mixtures thereof; wherein R¹ and R' are the same as above;
d) optionally, hydrogenating said mixture formed in step (c);
e) quaternizing said fabric softening active precursor mixture from step (b) by reacting said mixture with a quaternizing agent of the formula RX, wherein R is the same as defined in step (b) and X is a softener compatible anion, forming a fabric softening active having the formula: 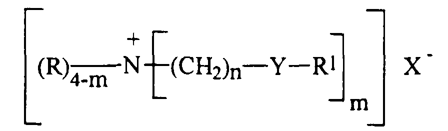 wherein R, R¹, X, Y, m, and n are the same as above;
f) mixing said fabric softening active with a principal solvent having a ClogP of from about 0.15 to about 0.64 thereby forming a fabric softener premix;
g) forming a water seat by combining water and a mineral acid; and
h) admixing said premix and said water seat with agitation to form a fabric softening composition;
provided at least step (c) is carried out in the presence of a chelating agent selected from the group consisting of diethylenetriaminepentaacetic acid, ethylenediamine-N,N'-disuccinnic acid, and mixtures thereof.

9. A process according to Claim 8 further comprising the step of adjusting the viscosity of said fabric softening composition with the addition of a solution of calcium chloride.

10. The process as claimed in either of Claims 8 or 9 further comprising the step of adding a low molecular weight water soluble solvent selected from the group consisting of: ethanol, isopropanol, propylene glycol, 1,3-propanediol, propylene carbonate, hexylene glycol, and mixtures thereof to said premix.

## Patentansprüche

1. Verfahren zur Herstellung eines Gewebeweichmacherwirkstoffs, die Schritte umfassend:
a) Bereitstellung einer Triglyceridquelle und Umsetzung dieser Triglyceridquelle unter Bildung einer Mischung, wobei diese Mischung Fettsäuren mit der Formel R¹CO₂H, Fettsäureester mit der Formel R¹CO₂R" oder Mischungen davon umfaßt: worin R¹ C₁-C₂₂-Alkyl, C₂-C₂₂-Alkenyl und Mischungen davon ist; R" C₁-C₄-Alkyl, C₂-C₄-Monohydroxyalkyl, C₃-C₄-Dihydroxyalkyl darstellt;
b) Umsetzung dieser Mischung aus Schritt (a) mit einem oder mehreren Aminen mit der Formel: 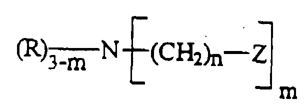 worin R C₁-C₆-Alkyl, C₁-C₆-Hydroxyalkyl, Benzyl oder Mischungen davon darstellt; Z jeweils unabhängig aus der Gruppe bestehend aus - OH, -CHR'OH, -CH(OH)CH₂OH, -NH₂ und Mischungen davon gewählt ist, worin R' C₁-C₄-Alkyl darstellt; m eine ganze Zahl von 1 bis 3 ist; n eine ganze Zahl von 1 bis 4 ist; unter Bildung einer Gewebeweichmacherwirkstoffvorstufenmischung mit der Formel: 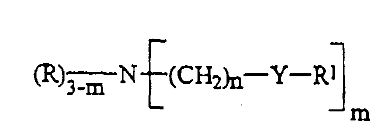 worin R, R¹, m und n dieselben wie oben sind: Y die Formel aufweist: 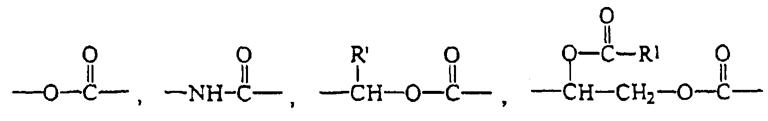 oder Mischungen davon; worin R¹ und R' dieselben wie oben sind; und
c) Quaternisierung dieser Gewebeweichmacherwirkstoffvorstufenmischung aus Schritt (b) durch Umsetzung dieser Mischung mit einem Quaternisierungsmittel der Formel RX, worin R genauso wie in Schritt (b) definiert ist und X ein weichmacherkompatibles Anion darstellt, wobei ein Gewebeweichmacherwirkstoff mit der Formel gebildet wird: 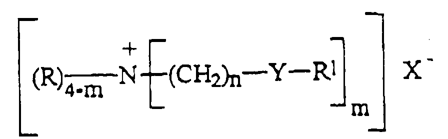 worin R, R¹, X. Y, m und n dieselben wie oben sind;
vorausgesetzt, daß mindestens Schritt (c) in Gegenwart eines Komplexbildners, ausgewählt aus der Gruppe bestehend aus Diethylentriaminpentaessigsäure, Ethylendiamin-N,N'-dibernsteinsäure und Mischungen davon, durchgeführt wird.

2. Verfahren nach Anspruch 1, worin der Umsetzungsschritt der Triglyceridquelle weiterhin die Umsetzung in Gegenwart des Komplexbildners einschließt und der Veresterungsschritt weiterhin die Veresterung in Gegenwart des Komplexbildners einschließt.

3. Verfahren nach Anspruch 1 oder 2, worin die Gesamtmenge an zu dem Verfahren hinzugegebenen Komplexbildner innerhalb des Bereichs von 10 ppm bis 5000 ppm bezogen auf das Gewicht an gebildetem Wirkstoff reicht.

4. Verfahren nach mindestens einem der Ansprüche 1-3, wobei die Triglyceridquelle ausgewählt ist aus der Gruppe bestehend aus Talg, teilweise hydriertem Talg, Schmalz, teilweise hydriertem Schmalz, Pflanzenölen, teilweise hydrierten Pflanzenölen und Mischungen davon.

5. Verfahren nach mindestens einem der Ansprüche 1-4, worin das Pflanzenöl oder das teilweise hydrierte Pflanzenöl ausgewählt ist aus der Gruppe bestehend aus Canolaöl, teilweise hydriertem Canolaöl, Safloröl, teilweise hydriertem Safloröl, Erdnußöl, teilweise hydriertem Erdnußöl, Sonnenblumenöl, teilweise hydriertem Sonnenblumenöl, Maiskeimöl, teilweise hydriertem Maiskelmöl, Sojaöl, teilweise hydriertem Sojaöl, Tallöl, teilweise hydriertem öl, Reisöl, teilweise hydriertem Reisöl und Mischungen davon.

6. Verfahren nach Anspruch 1, worin das Verfahren weiterhin den Schritt der Zugabe von 0.001 Gew.-% bis 2 Gew.-% bezogen auf den gebildeteten Wirkstoff einer Antioxidationsverbindung zu jedem oder allen der Schritte (a), (b) oder (c) umfaßt.

7. Verfahren zur Herstellung einer Gewebeweichmachervormischungszusammensetzung, die Schritte umfassend:
(a) Bereitstellung einer Trlglyceridquelle und die Umsetzung dieser Triglyceridquelle unter Bildung einer Mischung, wobei diese Mischung Fettsäuren mit der Formel R¹CO₂H, Fettsäureester mit der Formel R¹ CO₂R" oder Mischungen davon umfaßt; worin R¹ C₁-C₂₂-Alkyl, C₂-C₂₂-Alkenyl und Mischungen davon ist: R" C₁-C₄-Alkyl, C₂-C₄-Monohydroxyalkyl. C₃-C₄-Dihydroxyalkyl darstellt;
(b) Umsetzung dieser Mischung aus Schritt (a) mit einem oder mehreren Aminen mit der Formel: 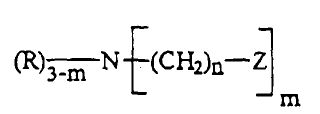 worin R C₁-C₆-Alkyl, C₁-C₆-Hydroxyalkyl, Benzyl oder Mischungen davon darstellt; Z jeweils unabhängig aus der Gruppe bestehend aus -OH, -CHR'OH, -CH(OH)CH₂OH, -NH₂ und Mischungen davon gewählt ist, worin R' C₁-C₄-Alkyl darstellt; m eine ganze Zahl von 1 bis 3 ist; n eine ganze Zahl von 1 bis 4 ist; unter Bildung einer Gewebeweichmacherwirkstoffvorstufenmischung mit der Formel: 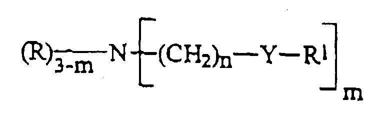 worin R, R¹, m und n dieselben wie oben sind; Y die Formel aufweist: 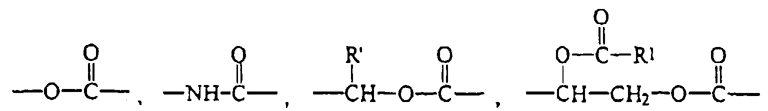 oder Mischungen davon: worin R¹ und R' dieselben wie oben sind;
c) Quaternisierung dieser Gewebeweichmacherwirkstoffvorstufenmischung aus Schritt (b) durch Umsetzung dieser Mischung mit einem Quaternisierungsmittel der Formel RX, worin R genauso wie in Schritt (b) definiert ist und X ein weichmacherkompatibles Anion darstellt, wobei ein Gewebeweichmacherwirkstoff mit der Formel gebildet wird: 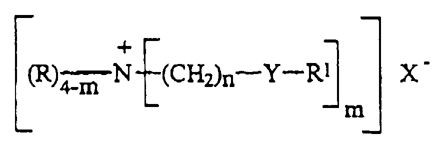 worin R, R¹, X, Y, m und n dieselben wie oben sind; und
d) Mischen dieses Gewebeweichmacherwirkstoffs mit einem Hauptlösungsmittel mit einem ClogP von 0,15 bis 0,64, wobei eine Gewebeweichmachervormischung gebildet wird;
vorausgesetzt, daß mindestens Schritt (c) in Gegenwart eines Komplexbildners, ausgewählt aus der Gruppe bestehend aus Diethylentriaminpentaessigsäure, Ethylendiamin-N,N'-dibernsteinsäure und Mischungen daraus, durchgeführt wird.

8. Verfahren zur Herstellung einer Gewebeweichmacherzusammensetzung, die Schritte umfassend:
a) Bereitstellung einer Triglyceridquelle und Umsetzung dieser Triglyceridquelle unter Bildung einer Mischung, wobei die Mischung Fettsäuren mit der Formel R¹CO₂H, Fettsäureester mit der Formel R¹CO₂R" oder Mischungen davon umfaßt: worin R¹ C₁-C₂₂-Alkyl, C₂-C₂₂-Alkenyl und Mischungen davon ist; R" C₁-C₄-Alkyl, C₂-C₄-Monohydroxyalkyl, C₃-C₄-Dihydroxyalkyl darstellt;
b) wahlweise Hydrierung der in Schritt (a) gebildeten Mischung;
c) Umsetzung der Mischung aus Schritt (a) mit einem oder mehreren Aminen mit der Formel: 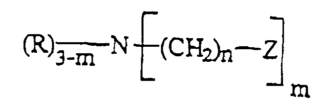 worin R C₁-C₆-Alkyl, C₁-C₆-Hydroxyalkyl, Benzyl oder Mischungen davon darstellt; Z jeweils unabhängig aus der Gruppe bestehend aus -OH,-CHR'OH, -CH(OH)CH₂OH,-NH₂ und Mischungen davon gewählt ist, worin R' C₁-C₄-Alkyl darstellt; m eine ganze Zahl von 1 bis 3 ist; n eine ganze Zahl von 1 bis 4 ist; unter Bildung einer Gewebeweichmacherwirkstoffvorstufenmischung mit der Formel: 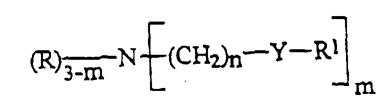 worin R, R¹, m und n dieselben wie oben sind; Y die Formel aufweist: 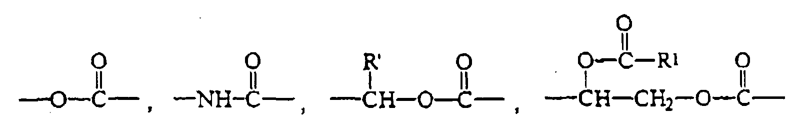 oder Mischungen davon; worin R¹ und R' dieselben wie oben sind;
d) wahlwiese Hydrierung der in Schritt (c) gebildeten Mischung;
e) Quaternisierung der Gewebeweichmacherwirkstoffvorstufenmischung aus Schritt (b) durch Umsetzung dieser Mischung mit einem Quaternisierungsmittel mit der Formel RX, worin R genauso wie in Schritt (b) definiert ist und X ein weichmacherkompatibles Anion darstellt, wobei ein Gewebeweichmacherwirkstoff mit der Formel gebildet wird: 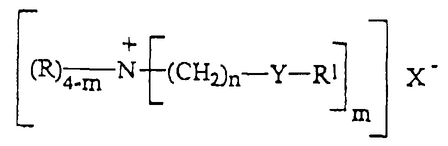 worin R, R¹, X, Y, m und n dieselben wie oben sind;
f) Mischen dieses Gewebeweichmacherwirkstoffs mit einem Hauptlösungsmittel mit einem ClogP von ungefähr 0,15 bis ungefähr 0,64, wobei eine Gewebeweichmachervormischung gebildet wird;
g) Bildung einer Wassergrundlage durch Kombinierung von Wasser und einer Mineralsäure; und
h) Zusammenmischen der Vormischung und der Wassergrundlage unter Rühren,wobei eine Gewebeweichmacherzusammensetzung gebildet wird;
vorausgesetzt, daß mindestens Schritt (c) in Gegenwart eines Komplexbildners, ausgewählt aus der Gruppe bestehend aus Diethylentriaminpentaessigsäure, Ethylendiamin-N,N'-dibernsteinsäure und Mischungen davon, durchgeführt wird.

9. Verfahren nach Anspruch 8, das weiterhin den Schritt der Einstellung der Viskosität der Gewebeweichmacherzusammensetzung mit der Zugabe einer Calciumchloridlösung umfaßt.

10. Verfahren nach Anspruch 8 oder 9, das weiterhin den Schritt der Zugabe eines wasserlöslichen Lösungsmittels mit niedrigem Molekulargewicht, ausgewählt aus der Gruppe bestehend aus : Ethanol, Isopropanol, Propylenglykol, 1,3-Propandiol, Propylencarbonat, Hexylenglykol und Mischungen davon, zu der Vormischung umfaßt.

## Revendications

1. Procédé pour préparer une substance active en tant qu'assouplissant textile comprenant les étapes consistant à :
a) obtenir une source de triglycéride et faire réagir ladite source de triglycéride pour former un mélange, ledit mélange comprenant des acides gras répondant à la formule R¹CO₂H, des esters d'acides gras répondant à la formule R¹CO₂R", ou des mélanges de ceux-ci ; où R¹ est un groupe alkyle en C₁-C₂₂, alcényle en C₂-C₂₂, et des mélanges de ceux-ci ; R" est un groupe alkyle en C₁-C₄, monohydroxyalkyle en C₂-C₄, dihydroxyalkyle en C₃-C₄ ;
b) faire réagir ledit mélange de l'étape (a) avec une ou plusieurs amines répondant à la formule : 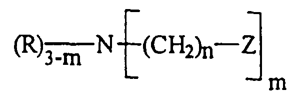 dans laquelle R est un groupe alkyle en C₁-C₆, hydroxyalkyle en C₁-C₆, benzyle, ou des mélanges de ceux-ci ; chaque Z est choisi indépendamment dans le groupe constitué par -OH, -CHR'OH, -CH(OH)CH₂OH, -NH₂, et des mélanges de ceux-ci, où R' est un groupe alkyle en C₁-C₄; m est un nombre entier de 1 à 3 ; n est un nombre entier de 1 à 4 ; pour former un mélange précurseur de substance active en tant qu'assouplissant textile répondant à la formule : 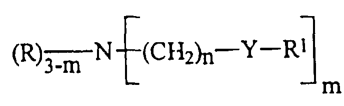 dans laquelle R, R¹, m et n sont les mêmes que ci-dessus ; Y répond à la formule : 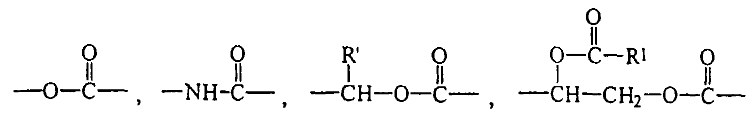 ou leurs mélanges ; où R¹ et R' sont les mêmes que ci-dessus ; et
c) quaterniser ledit mélange précurseur de substance active en tant qu'assouplissant textile de l'étape (b) en faisant réagir ledit mélange avec un agent de quaternisation de formule RX, dans laquelle R a la même définition que dans l'étape (b) et X est un anion compatible avec l'assouplissant, pour former une substance active en tant qu'assouplissant textile répondant à la formule : 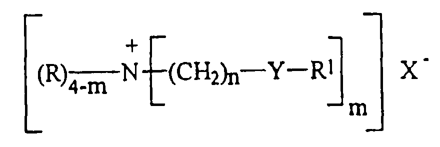
dans laquelle R, R¹, X, Y, m et n sont les mêmes que ci-dessus ; à la condition qu'au moins l'étape (c) soit réalisée en présence d'un agent chélatant choisi dans le groupe constitué par l'acide diéthylènetriamine-pentaacétique, l'acide éthylènediamine-N,N'-disuccinique, et des mélanges de ceux-ci.

2. Procédé selon la revendication 1, dans lequel ladite étape de réaction de ladite source de triglycéride inclut par ailleurs la réaction en présence dudit agent chélatant et ladite étape d'estérification inclut par ailleurs l'estérification en présence dudit agent chélatant.

3. Procédé selon l'une quelconque des revendications 1 ou 2, dans lequel la quantité totale d'agent chélatant ajoutée audit procédé se situe dans la gamme de 10 ppm à 5 000 ppm en poids, de la substance active formée.

4. Procédé selon l'une quelconque des revendications 1-3, dans lequel ladite source de triglycéride est choisie dans le groupe constitué par le suif, le suif partiellement hydrogéné, le saindoux, le saindoux partiellement hydrogéné, les huiles végétales, les huiles végétales partiellement hydrogénées, et des mélanges de ceux-ci.

5. Procédé selon l'une quelconque des revendications 1-4, dans lequel ladite huile végétale ou huile végétale partiellement hydrogénée est choisie dans le groupe constitué par l'huile de colza, l'huile de colza partiellement hydrogénée, l'huile de carthame, l'huile de carthame partiellement hydrogénée, l'huile d'arachide, l'huile d'arachide partiellement hydrogénée, l'huile de tournesol, l'huile de tournesol partiellement hydrogénée, l'huile de maïs, l'huile de maïs partiellement hydrogénée, l'huile de soja, l'huile de soja partiellement hydrogénée, le tallol, le tallol partiellement hydrogéné, l'huile de riz, l'huile de riz partiellement hydrogénée, et des mélanges de celles-ci.

6. Procédé selon la revendication 1, dans lequel ledit procédé comprend par ailleurs l'étape consistant à ajouter de 0,001 % à 2 % en poids, de la substance active formée, d'un composé antioxydant à l'une quelconque ou à la totalité des étapes (a), (b) ou (c).

7. Procédé pour préparer une composition de prémélange d'assouplissant textile, comprenant les étapes consistant à:
a) obtenir une source de triglycéride et faire réagir ladite source de triglycéride pour former un mélange, ledit mélange comprenant des acides gras répondant à la formule R¹CO₂H, des esters d'acides gras répondant à la formule R¹CO₂R", ou des mélanges de ceux-ci ; où R¹ est un groupe alkyle en C₁-C₂₂, alcényle en C₂-C₂₂, et des mélanges de ceux-ci ; R" est un groupe alkyle en C₁-C₄, monohydroxyalkyle en C₂-C₄, dihydroxyalkyle en C₃-C₄ ;
b) faire réagir ledit mélange de l'étape (a) avec une ou plusieurs amines répondant à la formule : 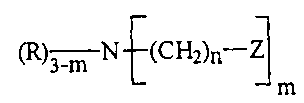 dans laquelle R est un groupe alkyle en C₁-C₆, hydroxyalkyle en C₁-C₆, benzyle, ou des mélanges de ceux-ci ; chaque Z est choisi indépendamment dans le groupe constitué par -OH, -CHR'OH, -CH(OH)CH₂OH, -NH₂, et des mélanges de ceux-ci, où R' est un groupe alkyle en C₁-C₄ ; m est un nombre entier de 1 à 3 ; n est un nombre entier de 1 à 4 ; pour former un mélange précurseur de substance active en tant qu'assouplissant textile répondant à la formule : 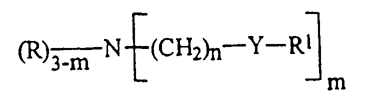 dans laquelle R, R¹, m et n sont les mêmes que ci-dessus ; Y répond à la formule : 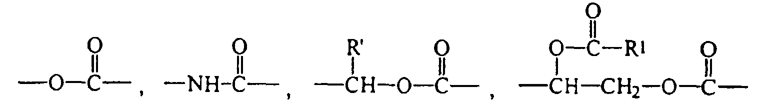 ou leurs mélanges ; où R¹ et R' sont les mêmes que ci-dessus ;
c) quaterniser ledit mélange précurseur de substance active en tant qu'assouplissant textile de l'étape (b) en faisant réagir ledit mélange avec un agent de quaternisation de formule RX, dans laquelle R a la même définition que dans l'étape (b) et X est un anion compatible avec l'assouplissant, pour former une substance active en tant qu'assouplissant textile répondant à la formule : 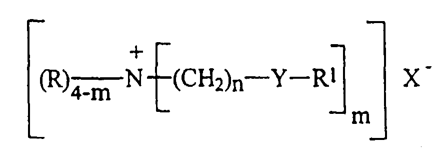 dans laquelle R, R¹, X, Y, m et n sont les mêmes que ci-dessus ; et
d) mélanger ladite substance active en tant qu'assouplissant textile avec un solvant principal ayant un ClogP de 0,15 à 0,64, pour former un prémélange d'assouplissant textile ;
à la condition qu'au moins l'étape (c) soit réalisée en présence d'un agent chélatant choisi dans le groupe constitué par l'acide diéthylènetriamine-pentaacétique, l'acide éthylènediamine-N,N'-disuccinique, et des mélanges de ceux-ci.

8. Procédé pour préparer une composition d'assouplissant textile comprenant les étapes consistant à :
a) obtenir une source de triglycéride et faire réagir ladite source de triglycéride pour former un mélange, ledit mélange comprenant des acides gras répondant à la formule R¹CO₂H, des esters d'acides gras répondant à la formule R¹CO₂R", ou des mélanges de ceux-ci ; où R¹ est un groupe alkyle en C₁-C₂₂, alcényle en C₂-C₂₂, et des mélanges de ceux-ci ; R" est un groupe alkyle en C₁-C₄, monohydroxyalkyle en C₂-C₄, dihydroxyalkyle en C₃-C₄ ;
b) le cas échéant, hydrogéner ledit mélange formé dans l'étape (a) ;
c) faire réagir ledit mélange de l'étape (a) avec une ou plusieurs amines répondant à la formule : 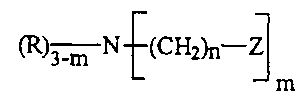 dans laquelle R est un groupe alkyle en C₁-C₆, hydroxyalkyle en C₁-C₆, benzyle, ou des mélanges de ceux-ci ; chaque Z est choisi indépendamment dans le groupe constitué par -OH, -CHR'OH, -CH(OH)CH₂OH, -NH₂, et des mélanges de ceux-ci, où R' est un groupe alkyle en C₁-C₄ ; m est un nombre entier de 1 à 3 ; n est un nombre entier de 1 à 4 ; pour former un mélange précurseur de substance active en tant qu'assouplissant textile répondant à la formule : 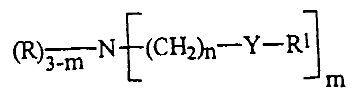 dans laquelle R, R¹, m et n sont les mêmes que ci-dessus ; Y répond à la formule : 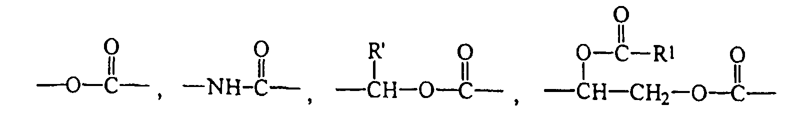 ou leurs mélanges ; où R¹ et R' sont les mêmes que ci-dessus ;
d) le cas échéant, hydrogéner ledit mélange formé dans l'étape (c) ;
e) quaterniser ledit mélange précurseur de substance active en tant qu'assouplissant textile de l'étape (b) en faisant réagir ledit mélange avec un agent de quaternisation de formule RX, dans laquelle R a la même définition que dans l'étape (b) et X est un anion compatible avec l'assouplissant, pour former une substance active en tant qu'assouplissant textile répondant à la formule : 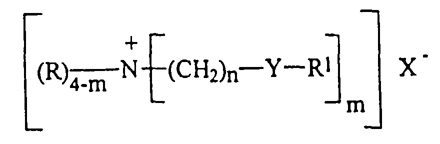 dans laquelle R, R¹, X, Y, m et n sont les mêmes que ci-dessus ;
f) mélanger ladite substance active en tant qu'assouplissant textile avec un solvant principal ayant un ClogP d'environ 0,15 à environ 0,64, pour former un prémélange d'assouplissant textile ;
g) former un fond d'eau en combinant de l'eau et un acide minéral ; et
h) mélanger ledit prémélange et ledit fond d'eau sous agitation pour former une composition d'assouplissant textile ;
à la condition qu'au moins l'étape (c) soit réalisée en présence d'un agent chélatant choisi dans le groupe constitué par l'acide diéthylènetriaminepentaacétique, l'acide éthylènediamine-N,N'-disuccinique, et des mélanges de ceux-ci.

9. Procédé selon la revendication 8, comprenant par ailleurs l'étape consistant à ajuster la viscosité de ladite composition d'assouplissant textile en ajoutant une solution de chlorure de calcium.

10. Procédé selon l'une quelconque des revendications 8 ou 9, comprenant par ailleurs l'étape consistant à ajouter audit prémélange un solvant soluble dans l'eau de faible masse moléculaire choisi dans le groupe constitué par l'éthanol, l'isopropanol, le propylèneglycol, le 1,3-propanediol, le carbonate de propylène, l'hexylèneglycol, et des mélanges de ceux-ci.
